# EUROPEAN PATENT SPECIFICATION

(11) **EP 2 384 206 B1**
(45) Date of publication and mention of the grant of the patent: **01.08.2018**
(21) Application number: 09805981.9
(22) Date of filing: 28.12.2009
(51) Int. Cl.: A61L 31/08, A61L 31/16, A61L 31/10

(54) **MEDICAL IMPLANTS AND METHODS OF MAKING MEDICAL IMPLANTS**
MEDIZINISCHE IMPLANTATE UND VERFAHREN ZUR HERSTELLUNG VON MEDIZINISCHEN IMPLANTATEN
IMPLANTS MÉDICAUX ET PROCÉDÉS DE FABRICATION ASSOCIÉS

(30) Priority: 26.12.2008 US 140911 P
(43) Date of publication of application: 09.11.2011
(73) Proprietor: Battelle Memorial Institute, Richland, WA 99352 (US)
(72) Inventor: SHAW, Wendy, J., Richland, WA 99352 (US); YONKER, Clement, R., Richland, WA 99352 (US); FULTON, John, L., Richland, WA 99352 (US); TARASEVICH, Barbara, J., Richland, WA 99352 (US); MCCLAIN, James, Richland, WA 99352 (US); TAYLOR, Doug, Richland, WA 99352 (US); MATSON, Dean W., Richland, WA 99352 (US)
(74) Representative: Wichmann, Hendrik
(86) International application number: PCT/US2009/069603
(87) International publication number: WO 2010/075590

(56) References cited:
- EP-A1- 0 982 041
- WO-A2-2005/069889
- US-A- 5 356 433
- US-A1- 2005 255 327
- US-A1- 2008 107 702
- K.MERRETT ET AL.: "Interactions of corneal cells with transforming growth factor beta2-modified poly dimethyl siloxane surfaces" JOURNAL OF BIOMEDICAL MATERIALS RESEARCH, PART A, vol. 67A, no. 3, 2003, - 2003 pages 981-993, XP002603606

## Description

### GOVERNMENT RIGHTS

This invention was made with Government support under contract DE-AC0676RL0-1830, awarded by the U.S. Department of Energy.

### TECHNICAL FIELD

The invention pertains to medical implant devices, implantable stent devices, and methods of making medical implant devices.

### BACKGROUND OF THE INVENTION

Many types of medical implant devices are commonly utilized in modern medicine. Some of these devices can have drawbacks such as causing allergic reaction or triggering blood clot formation. In implant devices such as stents, where the purpose of the device is to maintain an open artery or vein, the formation of a blood clot is contrary to the purpose of the device and can have negative effects which can even be life threatening.

Antithrombotic agents are of interest to utilize in conjunction with medical implant devices. Naturally occurring proteins that have antithrombotic or anticoagulation activity and can thereby alleviate blood clot formation include, for example, heparin. Heparin is known to act by binding to antithrombin III and inactivating thrombin. Recently, heparin has been utilized in conjunction with stents in attempt to prevent clot formation. However, heparin is mobile within the body and is able to diffuse away from the site of the stent. Accordingly, the effect of heparin under these circumstances is very short term. On the other hand, long term as well as short term effectiveness is desirable to deter clot formation and occlusion of the stent or blood vessel.
US5356433 A discloses a medical device having a biocompatible surface being made by a specific process comprising the steps of:
providing a metallic member having a tantalum oxide-containing surface thereof, said metallic member being flexible and being sized and shaped as a stent;
treating the tantalum oxide containing surface with an amino-terminated organosilane having amine reactive sites and covalently linking the organosilane with the flexible metallic member to effect a condensation reaction that covalently links the tantalum oxide and silicon moieties of the organosilane in order to form a flexible organosilane-coated tantalum stent; applying a biologically active agent compostion to the organosilane-coated tantalum stent and forming a covalent linkage between the organosilane and the biologically active agent to provide a coated flexible tantalum stent; and the coated flexible tantalum stent thus formed has a coating which withstands cracking upon flexure, and the tantalum stent thus formed is a biocompatible tantalum stent which, when implanted within a living body, prevents substantial thrombus from occurring on its surface while not significantly interfering with endothelialization of said surface.
US2005/0255327 A1 discloses an article comprising a bioactive surface, said article comprising: a) a substrate; b) an aminopropyltrimethoxysilane (APTMS) layer on said substrate, wherein said layer is of substantially uniform thickness and is substantially defect free; and c) a biologically functional molecule covalently attached to said APTMS.
EP 0982041 A1 discloses a process for coating a medical device with a silane or siloxane having at least one functional group selected from the group consisting of an isocyanate, an isothiocyanate, an ester, an anhydride, an acyl halide, an alkyl halide, an epoxide and an aziridine, comprising plasma depositing said silane or said siloxane.
US20080107702 A1 discloses a method for the immobilization of mediator molecules on implant materials, characterized in that in a first step anchor molecules are covalently bound to the surface of the implant material, wherein these anchor molecules have functional groups to which further chemical compounds can be covalently bound, and in a second step mediator molecules can be immobilized on the implant material via these functional groups.
WO2005069889 A2 discloses a method of preparing a functional surface, said method comprising: providing a substrate; providing an effective amount of active component comprising a functional group, spacer group, and a binding group, wherein said functional group comprises one or more terminal hydroxyl groups; providing an effective amount of cross-linking component; providing an effective amount of matrix-forming component; and affixing said active component, said cross-linking component and said matrix-forming component onto said substrate, thereby forming a hydroxyl functional surface.

It is desirable to develop alternative implant devices and methods of forming implant devices to address these issues.

### SUMMARY OF THE INVENTION

The invention pertains to a medical implant device as defined in the claims.

In one aspect, the invention as defined in the claims pertains to an implantable stent device comprising a stent core having an oxidized surface with a layer of silane derivative covalently bonded to the oxidized surface. A spacer layer as defined in the claim comprising polyethylene glycol **(PEG)** is covalently bonded to the layer of silane derivative and a protein is covalently bonded to the **PEG.**

In one embodiment the surface may comprise at least one oxidized site wherein the at least one silane derivative is covalently bonded to the at least one oxidized site.

In one embodiment the surface is a polymer surface having a moiety forming a covalent bond with the silane derivative. The silane derivative and the polymer surface may be coupled through an organosilane linkage, for example a dialkoxysilane or a monoalkosysilane. The surface may comprise a natural polymer selected from cellulose, starch (amylose and amylopectin), proteins, silk, spider webs, polyhydroxyalkanoates, deoxyribonucleic acid (DNA), natural rubber, and polysacharides.

Disclosed is a stent which comprises a plurality of layers that form a laminate coating on said stent; wherein at least one of said layers comprises a bio-absorbable polymer and at least one of said layers comprises one or more active agents; wherein at least a portion of the active agent is in crystalline form wherein the active agent is the same or different from the biological agent covalently bonded to the silane derivative moieties.

### BRIEF DESCRIPTION OF THE DRAWINGS

Preferred embodiments of the invention are described below with reference to the following accompanying drawings.
Fig. 1 is a cross-sectional side view of an implant device (not according to the invention).
Fig. 2 is a cross-sectional side view of an implant device in accordance with another aspect of the invention.
Fig. 3 is a cross-sectional side view of an implant device (not according to the invention).
Fig. 4 is a cross-sectional side view of an implant device (not according to the invention).
Fig. 5 is a cross-sectional side view of an implant device (not according to the invention).
Fig. 6 is a cross-sectional side view of an implant device at an initial processing stage in accordance with one aspect of the invention.
Fig. 7 is a cross-sectional side view of an implant device at a processing stage subsequent to that depicted in Fig. 6.
Fig. 8 is a cross-sectional side view of an implant device at a processing stage subsequent to that depicted in Fig. 7.
Fig. 9 is a cross-sectional side view of an implant device (not according to the invention) at a processing stage subsequent to that depicted in Fig. 8.
Fig. 10 is a cross-sectional side view of an implant device shown at an alternative processing step relative to Fig.9.
Fig. 11 is a cross-sectional side view of an implant device shown at a processing stage subsequent to that depicted in Fig. 10.

### DETAILED DESCRIPTION OF THE PREFERRED EMBODIMENTS

Medical implant devices are common in modern medicine in the treatment of a wide variety of conditions. However, implants can trigger biological problems and have side effects such as rejection of the device, allergic reaction and blood clot formation that can lead to thrombosis.

Modern implants include, for example, catheters, electrodes, stents, leads, pacemakers, cardioverter or defibrillator housings, artificial joints, screws, rods, ophthalmic implants, pins, bone plates, grafts, anastomotic devices, perivascular wraps, staples, shunts, dialysis grafts, colostomy bag attachment devices, ear drainage tubes, vertebral disks, suture anchors, hemostatic barriers, clamps, plates, clips, vascular implants, tissue scaffolds, dressings, bone substitutes, intraluminal devices and vascular supports, to name but a few. In the case of stents and similar devices, the side effect of blood clot formation can lead to occlusion of the stent or the blood vessel in which it is inserted, and can even lead to death.

Described herein are implant devices and method of producing such devices, which have one or more bioactive agents covalently bound to the implant device to alleviate negative side effects.

As utilized herein, the term "bioactive agent" refers to a molecule that has biological activity. A bioactive agent can be synthetic or naturally occurring and includes but is not limited to biopolymers (peptides, proteins, nucleic acids), amino acids, pharmaceutical agents, and small organic molecules.

The term "compressed fluid" as used herein refers to a fluid of appreciable density (e.g. >2g/cc) that is a gas at standard temperature and pressure. "Supercritical fluid", "near critical fluid", "near-supercritical fluid", "critical fluid", "densified fluid", or "densified gas" as utilized herein refers to a compressed fluid under conditions wherein the temperature is at least 80% of the critical temperature of the fluid and the pressure is at least 50% of the critical pressure of the fluid.

Examples of substances that demonstrate supercritical or near critical behavior suitable for the present invention include, but are not limited to carbon dioxide, isobutylene, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfluoropropane, chloroform, trichloro-fluoromethane, dichlorodifluoromethane, dichloro-tetrafluoroethane) and mixtures thereof.

"Sintering" as used herein refers to the process by which parts of the matrix or the entire polymer matrix becomes continuous (e.g., formation of a continuous polymer film). As discussed below, the sintering process is controlled to produce a fully conformal continuous matrix (complete sintering) or to produce regions or domains of continuous coating while producing voids (discontinuities) in the matrix. The sintering process is controlled such that some phase separation is obtained between different polymers (e.g., polymers A and B) and/or to produce phase separation between discrete polymer particles. The adhesion properties of the coating are improved to reduce flaking or detachment of the coating from the substrate during manipulation in use through the sintering process. As described below, in some embodiments, the sintering process is controlled to provide incomplete sintering of the polymer matrix. In embodiments involving incomplete sintering, a polymer matrix is formed with continuous domains, and voids, gaps, cavities, pores, channels or interstices that provide spaces for sequestering therapeutic agents which can be released under controlled conditions. Depending on the nature of the polymer, the size of polymer particles and/or other polymer properties, a compressed gas, a densified gas, a near critical fluid or a super-critical fluid may be employed. In one example, carbon dioxide is used to treat a substrate that has been coated with a polymer and a drug, using dry powder and rapid expansion of supercritical solutions (RESS) electrostatic coating processes. In another example, isobutylene is employed in the sintering process. In other examples a mixture of carbon dioxide and isobutylene can be utilized.

When an amorphous material is heated to a temperature above its glass transition temperature, or when a crystalline material is heated to a temperature above a phase transition temperature, the molecules of the material are more mobile, which in turn means that they are more active and thus more prone to reaction such as oxidation. However, when an amorphous material is maintained at a temperature below its glass transition temperature, the amorphous molecules are substantially immobilized and thus less prone to reactions. Likewise, when a crystalline material is maintained at a temperature below its phase transition temperature, the crystalline molecules are substantially immobilized and thus less prone to reaction. Accordingly, processing drug components at mild conditions, such as the deposition and sintering conditions described herein, minimizes cross-reaction and degradation of the drug component. One type of reaction that is minimized by the processes of the invention relates to the ability to avoid conventional solvents which in turn minimizes auto-oxidation of the drug, whether in amorphous, semi-crystalline, or crystalline form, by reducing exposure thereof to free radicals, residual solvents and auto-oxidation initiators.

"Rapid Expansion of Supercritical Solutions" (RESS) as used herein involves the dissolution of a polymer into a compressed fluid, typically a supercritical fluid, followed by rapid expansion into a chamber at lower pressure, typically near atmospheric conditions. The rapid expansion of the supercritical fluid solution through a small opening, with its accompanying decrease in density, reduces the dissolution capacity of the fluid and results in the nucleation and growth of polymer particles. The atmosphere of the chamber is maintained in an electrically neutral state by maintaining an isolated "cloud" of gas in the chamber. Carbon dioxide or another appropriate gas employed to prevent electrical charge is transferred from the substrate to the surrounding environment.

"Bulk properties" of a coating that include a pharmaceutical or a biological agent which can be enhanced through the methods of the invention include for example: adhesion, smoothness, conformallity, thickness, and compositional mixing.

"Electrostatically charged", "electrical potential" or "electrostatic capture" as used herein refers to the collection of the spray-produced particles upon a substrate that has a different electrostatic potential than the sprayed particles. Thus, the substrate is at an attractive electronic potential with respect to the particles exiting, which results in the capture of the particles upon the substrate (i.e. the substrate and particles are oppositely charged and the particles transport through the fluid medium of the capture vessel onto the surface of the substrate is enhanced via electrostatic attraction). This may be achieved by charging the particles and grounding the substrate or conversely charging the substrate and grounding the particles, or by some other process, which would be easily envisaged by one of skill in the art.

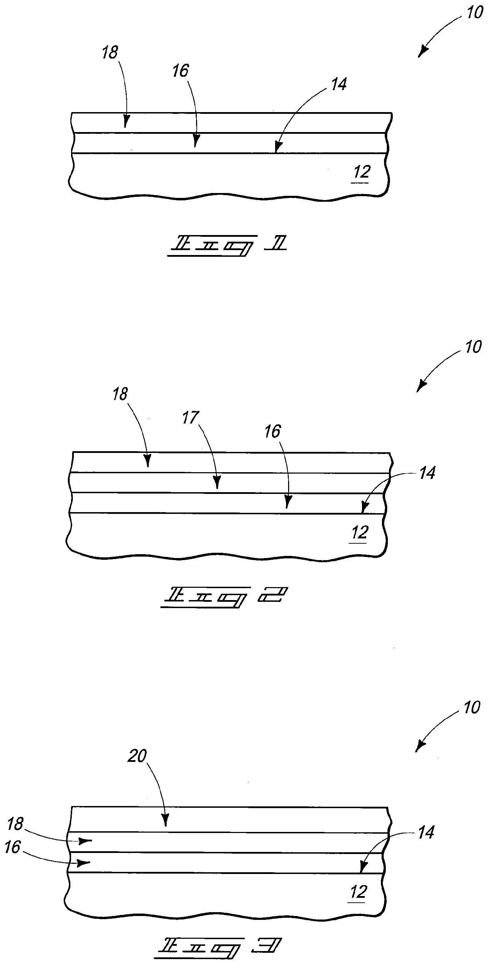

General aspects are illustrated and described with reference to Figures 1- 11. Referring initially to Fig. 1, an example implant device 10 is illustrated. Implant device 10 can be any of the devices listed above and in particular aspects can be a stent. Device 10 has a substrate 12 having an outer surface 14. The implant device can comprise materials such that surface 14 comprises one or more of stainless steel, cobalt chromium alloy, or alternative metallic alloys. Example cobalt chromium alloys include but are not limited to 0.05-0.15 weight % C, 1.00-2.00 weight % Mn, 0.040 weight % Si,
0.030 weight% P, 0.3 weight% S, 19.0-21.0 weight% Cr, 9.00-11.00 weight% Ni, 14.00-16.00 weight% W, 3.00 weight% Fe and the balance Co. Alternative alloy materials include but are not limited to 0.025 weight % maximum C, 0.15 weight% maximum Mn, 0.15 weight% maximum Si, 0.015 weight% maximum P, 0.01 weight% maximum S, 19.00-21.00 weight% maximum Cr, 33-37 weight % Ni, 9.0-10.5 Mo, 1.0 weight% maximum Fe, 1.0 weight% maximum Ti, and the balance Co.

A layer 16 of silane material or alternative polymer material is disposed on surface 14. Layer 16 can be formed of a silane derivative that covalently bonds to surface 14 after oxidation of surface 14 (described below) or can be a polymer material having a silane derivative covalently bonded thereto through an organosilane linkage. The organosilane linkage can be either a dialkoxysilane silane or a monoalkoxysilane. The polymer can be either natural or synthetic. Natural polymers can include, for example cellulose, starch (amylase and amylopectin), proteins, silk, spider webs, polyhydroxyalkanoates, deoxyribonucleic acid (DNA), natural rubber and polysaccharides.

Where the substrate surface is stainless steel, the covalent attachment can be formed between oxidized Fe or oxidized Cr on surface 14 and Si of the silane material to form Fe-O-Si-R or Cr-O-Si-R. Covalent attachment of layer 16 to the implant substrate can advantageously inhibit or prevent loss of the layer, and any subsequently bound layers, during the life of the implant.

A layer 18 of bioactive agent is on and covalently attached to the silane material layer 16. The bioactive agent is defined in the claims and can be an agent targeted to alleviate the potential side effects of the particular implant device at issue. Where implant device 10 is a stent, layer 18 can comprise an antithrombogenic agent such as heparin. It is to be understood that layer 18 is not limited to a single bioactive agent and can comprise two or more bioactive agents simultaneously, each being covalently bound to the silane material.

Where the bioactive agent is a protein it can be preferable that the protein be bonded to the silane derivative of the silane material through a peptide bond. Accordingly, the silane derivative will be chosen to have appropriate exposed amino groups or carboxylic acid groups. When heparin is utilized as the bioactive agent, it can be preferable for the silane derivative to have terminal amino groups which form a peptide bond with a carboxyl group of the heparin molecule. Numerous silane derivatives having terminal amino groups are available for purposes of the present invention. One example of such a silane derivative is aminopropyltriethoxysilane (APTES). Alternatively, layer 16 can be formed of polymer material such as polyacrylamide, polyvinylamine, copolymers of polyacrylamide, polyvinyl amine and ethyleneimide, copolymers of isopropylacrylamide and polyvinylamine, copolymers of isopropylacrylamide and N-(3-aminopropyl)methacrylamide or other polymer or copolymer containing NH2 groups.

The thickness of layer 16 can vary and can be controlled by varying the reaction time and/or the silane concentration, as can the number of reactive NH2 sites. Accordingly, high coverage of bioactive agent can be achieved by increasing the number of active NH2 sites.

The invention is described with reference to Fig. 2. As depicted, an intervening layer 17 is present between the silane material layer 16 and the bioactive agent 18. Layer 17 is a spacer material as defined in the claims comprising spacer molecules that are covalently bound to the silane derivative and are covalently bound to the bioactive agent. The spacer is a hetero-bifunctional polyethylene glycol (PEG) spacer. Utilization of PEG as spacer molecules has the benefit of allowing greater access to the biological agent by interacting molecules since there is less steric hindrance. Additionally, PEG reduces non- specific adsorption of proteins on the surface that can lead to thrombic events.

The PEG derivative is heterobifunctional, with a first reactive group on a first end for reacting with a surface group on the silane material. A second reactive group on the second end will preferably be capable of covalently bonding with the particular bioactive agent being utilized. Where the bioactive agent is heparin or another protein or peptide, the PEG derivative has a second reactive group comprising an amine group for forming a peptide bond with the bioactive agent. The silane derivative comprises terminal amine groups, and the PEG first reactive group comprises a reactive carboxyl group to form a peptide bond to the silane. Many such hetero- bifunctional PEG derivatives are available for use as linkers in the present application. Examples include but are not limited to COOH(CH2CH2O)ₙNH₂, where n equals an integer.

The use of covalently bound active agents can increase the active life of the agent relative to non-covalently bound agents since the agent is unable to migrate away from the implant device where the activity is desired. Utilizing heparin as an example, when covalently bonded to a stent utilizing a silane material or silane/PEG linker, the active life of the heparin can be extended to up to 52 weeks or longer from the onset of activity (see below).

In an alternative aspect disclosed herein, the implant having the covalently bound bioactive agent can be coated as depicted in Figs. 3-5. As shown in Fig. 3, a coating layer 20 has been provided over bioactive agent layer 18.

Although shown as discreet layers, it is to be understood that coating layer 20 can overlap or partially overlap layer 18. Layer 20 can preferably comprise one or more bio- absorbable polymer materials that are capable of being degraded by the organism into which the implant is placed. Example bio-absorbable polymer materials that may be utilized include but are not limited to poly(lactide-co-glycolide (PLGA); poly(dl-lactide) (DLPLA); poly(l-lactide) (LPLA); polyglycolide (PGA); poly(dioxanone) (PDO); poly(glycolide-co-trimethylene carbonate) (PGA- TMC);
poly(l-lactide-co-glycolide) (PGA-LPLA); poly(dl-lactide-co-glycolide) (PGA-DLPLA); poly(l-lactide-co-dl-lactide) (LPLA-DLPLA); or poly(glycolide-co- trimethylene carbonate-co-dioxanone) (PDO-PGA-TMC). When the biopolymer includes PLGA, the PLGA can have a molecular weight of from about 29 kD to about 90 kD.

It is to be understood that the bio-absorbable polymer layer can comprise mixtures of polymer materials and/or polymer and copolymer materials. For instance, the layer can contain at least two polymers and can comprise a first PLGA copolymer at a ratio of about 60:40 and a second PLGA copolymer at a ratio of from about 70:30 to about 90:10.

The bio-absorbable polymer layer is an over-coating relative to the underlying bioactive agent. Thus the polymer layer can delay the activity of the bioactive agent until some or all of the polymer material has been removed from the implant.

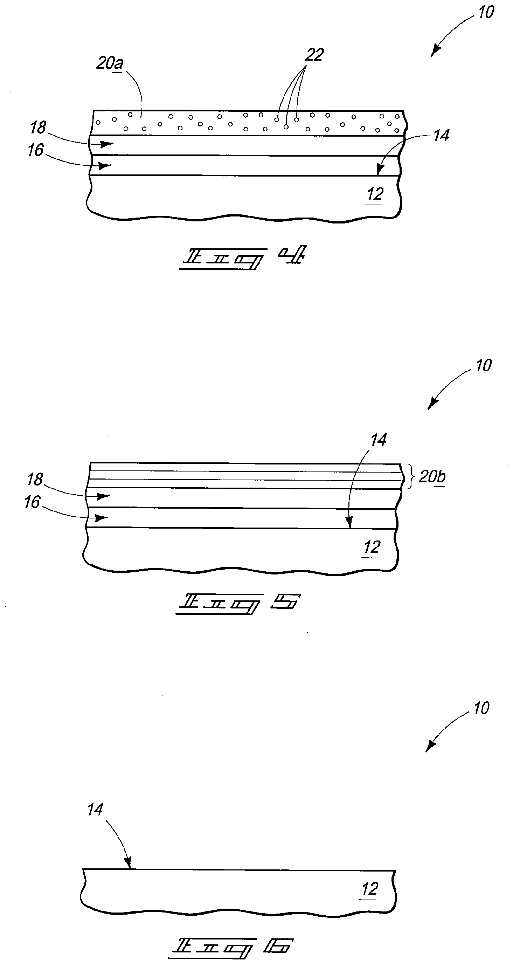

Referring next to Fig. 4, such shows an alternative form of coating layer 20. In such embodiment, layer 20 can preferably comprise at least one bio-absorbable polymer, as described above, and at least one pharmaceutical compound. Example classes of pharmaceutical compounds which can be utilized individually or in combination include but are not limited to immunosuppressive drugs, antirestenotic agents (e.g., paclitaxel), antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Grahn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, antigens, beta-receptor blockers, non-steroidal antiinflammatory drugs {NSAIDs], cardiac glycosides, acetylsalicylic acid, virustatics, aclarubicin, acyclovir, cisplatin, actinomycin, alpha- and beta- sympatomimetics, (dmeprazole, allopurinol, alprostadil, prostaglandins, amantadine, ambroxol, amlodipine, methotrexate, S-aminosalicylic acid [sic], amitriptyline, amoxicillin, anastrozole, atenolol, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenytoin, riluzoles, risedronate, sildenafil, topiramate, macrolide antibiotics, oestrogen and oestrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tetracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobuterol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorelbine, vinpocetine, viquidil, warfarin, xantinol nicotinate, xipamide, zafirlukast, zalcitabine, zidovudine, zolmitriptan, zolpidem, zoplicone, and zotipine.

Example antistenotic drugs that can be utilized include but are not limited to paclitaxel and paclitaxel derivatives.

Example immunosuppressive drugs which can be utilized include but are not limited to macrolide immunosuppressive (limus) drugs selected from the group consisting of rapamycin (sirolimus), 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl- rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin, 40-O-(2- Hydroxy)ethoxycar-bonylmethyl-rapamycin, 40-O-(3-Hydroxy)propyl-rapamycin, 40-O-(6-Hydroxy)hexyl-rapamycin, 40-O-[2-(2-Hydroxy)ethoxy]ethyl-rapamycin, 40-O-[(3S)-2,2-Dimethyldioxolan-3-yl]methyl-rapamycin, 40-O-[(2S)-2,3-Dihydroxyprop-1-yl]-rapamycin, 40-O-(2-Acetoxy)ethyl-rapamycin, 40-O-(2-Nicotinoyloxy)ethyl-rapamycin, 40-O-[2-(N-Morpholino)acetoxy]ethyl-rapamycin, 40-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene- rapamycin, (26R)-26-Dihydro-40-O-(2-hydroxy)ethyl-rapamycin, 28-O-Methyl-rapamycin, 40-O-(2-Nicotinamidoethyl)-rapamycin, 40-O-(2-Acetaminoethyl)-rapamycin, 40-O-(2-Tolyisulfonamidoethyl)-rapamycin, 40-O-(2-(N-Methyl-imidazo-2'-ylcarbethoxamido)ethyl)-rapamycin, 40-O-(2-Aminoethyl)-rapamycin, 40-O-(2-Ethoxycarbonylaminoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus), including prodrugs, derivatives, analogs, hydrates and salt forms thereof.

The pharmaceutical agents can be within the layer of polymer as shown, or alternatively form a separate layer either beneath or over the polymer layer, or partially overlapping the polymer layer (not shown). In particular aspects the pharmaceutical agent can be held to the implant by physisorption. It can be preferable that at least 50% of the pharmaceutical be in crystalline form. The presence of the pharmaceutical in crystalline form can be confirmed by, for example, the x-ray spectrum, the Raman spectrum, the Differential Scanning Calorimetry (DSC) curve, the Wide Angle X-ray Scattering (WAXS) spectrum, the wide angle radiation scattering spectrum or the Infra Red (IR) spectrum.

Elution of the pharmaceutical agent into the body will occur over a period of time. It can be preferable that the pharmaceutical elution profile be such that about 10% to about 50% of at least one pharmaceutical agent elute within one week after implantation of the device (under physiological conditions). About 25% to about 75% of at least one pharmaceutical preferably elutes within two weeks and from about 50% to about 100% is eluted after about eight weeks. The amount and type of bio-absorbable polymer present can affect the elution profile of the pharmaceutical agent(s).

Referring to Fig. 5, layer 20 comprises two or more layers. Such layering can comprise, for example, one or more layer of absorbable polymer materials which can be of the same composition or can differ in composition. Such layering can further comprises one or more layer of pharmaceutical agent(s), where the composition of the pharmaceutical agent layers can be the same or can differ. As depicted in Fig. 5, layer 20 can comprise three layers. However, it is to be understood that layer 20 can comprise as few as two layers or can comprise greater than three layers.

In one particular embodiment where layer 20 is made up of multiple layers, layer 20 can comprise a first layer of bio-absorbable polymer, a second layer of pharmaceutical agent over the first layer, a third layer of bio-absorbable polymer over the second layer, a fourth layer of pharmaceutical agent over the third layer and an outer fifth layer of bio-absorbable polymer over the fourth layer. The first, third and fifth polymer layers can be the same or can differ, and the second and fourth pharmaceutical agent layers can be the same or can differ. In a specific example, the first, third and fifth polymer layers can comprise PLGA and the second and fourth pharmaceutical layers can comprise rapamycin.

The presence of layer 20, in any of the embodiments depicted in the figures, can serve as a coating and can temporarily inhibit or prevent activity of the underlying bioactive agent until enough of the bio-absorbable polymer has been absorbed to allow access to the bioactive agent. The coating can block the activity of the bioactive agent such that initiation of activity of the bioactive agent occurs after some time delay post implant. The delay of initiation of activity can be, for example, about three weeks.

Once the activity of the covalently bound bioactive agent occurs, the activity is sustained over a relatively long period of time as compared to non- covalently bound agent. In the case of heparin, the activity initiates approximately three weeks after implant (if a polymer coating is present) and is sustained at an effective level for at least 90 days. In particular applications, heparin's activity can be sustained for at least 120 days, 200 days or ever 52 weeks or longer.

The surface morphology of the implant can also play a role in both foreign body (negative) and healing (positive) responses. Nano-textured surfaces can be utilized for purposes of the present invention. These surfaces are created by including particles of Ir02, TiO2, SiO2 or hydroxyapatite and can be utilized to promote new tissue in a controlled positive manner. The underlying morphology/topology of the implant surface revealed after bio-absorption of the coating can be purposely designed to provide a clinical effect beyond that of a bare, smooth metal surface. These effects include promotion of cell adhesion and antithrombotic effects.

Methods of producing implants of the invention are described with reference to Figs. 6-11. Referring initially to Fig. 6, a substrate 12 of an implant device 10 to be constructed is provided. The implant device can be any of those implant devices indicated above. For ease of description, the illustrated implant will be referred to as a stent, although such is not intended to in any way limit the scope of the invention. Fabrication of substrate 12 is typically complete in the entirety with the exception of the added coating(s) of the invention.

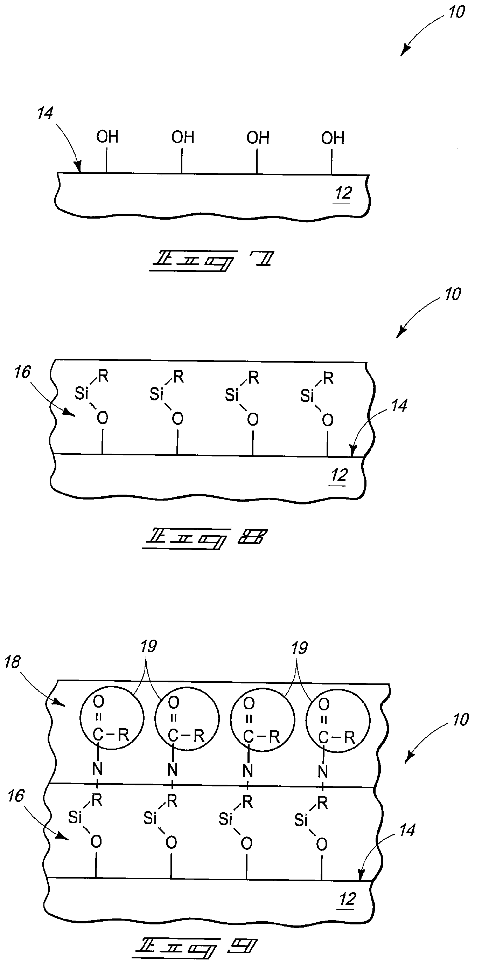

Substrate 12 is treated with an acid or base to clean and oxidize surface 14, producing a hydroxylated surface as depicted in Fig. 7. Referring to Fig. 8, oxidized surface 14 is silinated utilizing a silane derivative having one or more terminal amine to produce a silane layer 16 that is covalently bonded to the substrate and has available amino groups for further reaction. There are numerous silane derivatives available for purposes of the silanation reaction set forth herein. An example silane derivative that can be utilized is aminopropyltriethoxysilane. This derivative can be reacted in toluene or an alternative appropriate organic solvent to silanate the oxidized surface of device 10.

After depositing silane layer 16, a desired bioactive agent 19 as defined in the claims can be bonded to the available reactive amino groups of the silane layer as shown in Fig. 9. Where the bioactive agent is a protein, the amine group of the silane derivative can be reacted to form a peptide bond. Reaction to form a peptide bond can be conducted in the presence of a carbodiimide in an appropriate buffer. For example, heparin can be covalently bonded through a peptide bond to the amine group of the silane derivative in the presence of 1-ethyl-3-(3-dimethylaminopropyl)
carbodiimide hydrochloride (EDC) and sulfo-N- hydroxysuccinimide in 0.1 M 2-(N-morpholino)ethanesulfonic acid (MES) buffer.

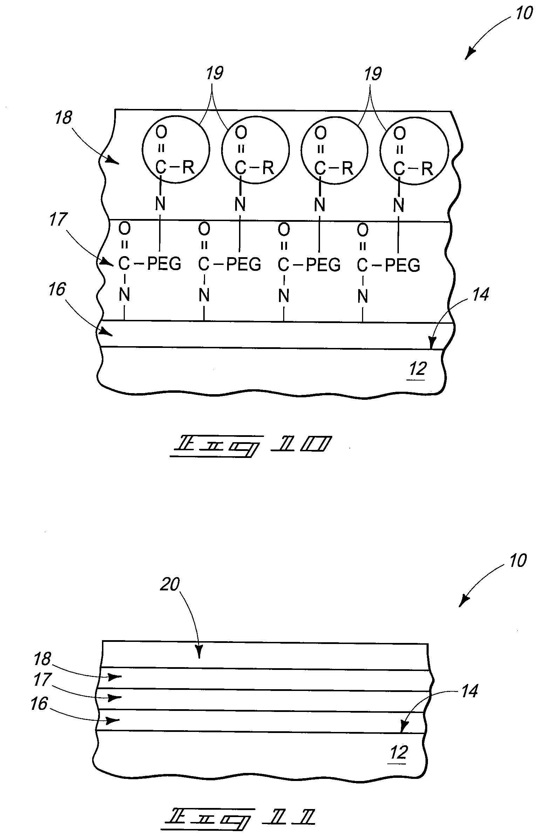

As shown in Fig. 10, the derivatized silane can alternatively be reacted with a hetero-bifunctional PEG to covalently attach the PEG to the silane layer. This reaction can be achieved by using similar condensation chemistry to that described above, and utilizing a carboxylated PEG. Once the PEG is covalently bound, the bioactive agent can be bonded to the hetero-bifunctional PEG by utilizing an amine group on the PEG and again performing a condensation reaction, as described above.

The implant having covalently bound bioactive agent can be utilized as is or can be further coated as described below with reference to Fig.
11. Although Fig. 11 depicts coating 20 as being a single layer, it is to be understood that such layer is representative of the embodiments discussed above and that coating 20 can comprise a single layer, two or more laminated layers, overlapping layers of polymer and pharmaceutical agent, etc. as set forth above.

Coating 20 can comprise at least one layer of bio-absorbable polymer. Example polymer materials are set forth above. A layer of bio-absorbable polymer material can be deposited utilizing a variety of methods. Preferred methods included but are not limited to Electrostatic Rapid Expansion of Supercritical Fluid Solutions (RESS), or Solution Enhanced Dispersion of Supercritical Fluids (SEDS). Alternative methods may include but are not limited to dip coating, vapor deposition and aerosol spray deposition.

Where RESS is utilized, the process can be as set forth in U.S. Patent No. 6,756,084, issued to Fulton et al. Utilizing the RESS process, densified gases in supercritical or liquid state are attractive media for the delivery of solid polymer material to be deposited in thin conformal films. Densified gas such as carbon dioxide or low molecular weight fluorinated hydrocarbons can be utilized as solvent. Rapid expansion of the solutions containing the dissolved solids from pressure or temperature conditions at which the solvents are in their supercritical fluid state through a small orifice or restrictor results in the formation of nanometer-scale solute particles suspended in a gaseous solvent stream.

To enhance RESS-generated nanoparticle collection efficiency on the surface of the implant, the RESS particles are charged during their formation utilizing an electric field applied to the tip of the expansion nozzle, or a secondary corona discharge in the presence of the nanoparticles, known as electrostatic RESS. Alternatively, the implant to be coated can be electrostatically charged to attract the nanoparticles in the RESS stream. The charged nanoparticles are thus attracted to the implant surface due to the electric field gradient, thus generating a uniform coating of polymer material. Due to the small size of the RESS particles, even implants having complex geometries, such as medical stents, can be coated uniformly and completely.

Once the RESS nanoparticles have been deposited, the newly formed layer is subjected to a sinter procedure to turn the RESS-deposited particles into a film. The sintering method utilized is based upon methodology disclosed in U.S. Patent No. 6,794,902, issued to Yonker et al. In the sintering process, the particle-covered implant surface is contacted with a supercritical fluid under conditions sufficient for forming a continuous film from the polymer particles. The particles may have a particle size of less than or equal to about one micron. The method may be performed by providing a pressure vessel that can contain a compressible fluid. The implant having RESS-deposited particles is provided within the vessel and the compressible fluid is maintained at supercritical or sub-critical (but non-liquid) state sufficient for forming a film from the deposited polymer particles. The glass
transition temperature (Tg) of the particles is reduced by subjecting the particles to the sintering conditions. This sintering method is advantageous in that thermally liable drugs and materials can be treated without loss of bioactivity.

SEDS can be utilized to form a layer of pharmaceutical. This method involves providing an aqueous solution of the pharmaceutical and decreasing the solvating power of the water by saturating it with carbon dioxide under supercritical conditions. The drug solution and a stream of supercritical carbon dioxide can be mixed utilizing a coaxial nozzle. The high velocity, turbulent, supercritical fluid stream breaks up the aqueous solution into very small droplets. A third stream containing an organic solvent can be utilized to overcome immiscibility problems between the aqueous and supercritical carbon dioxide phases. The three-nozzle process minimizes the amount of time that pharmaceuticals such as proteins, for instance, are exposed to denaturing conditions. Control over the size of the particle can be achieved by variation of process variables such as flow rates of the three input streams and pressure drop across the nozzle.

The drug coating can alternatively be applied by the electrostatic RESS process described above or by electrostatic deposition (eSTAT). Utilizing the eSTAT method, the pharmaceutical agent is micronized through milling, preserving the crystallinity of the agent. The micronized agent powder is dispersed into the coating system utilizing a pulse of C02 gas. The finely dispersed drug powder is then electrostatically deposited on the implant substrate utilizing the same mechanism as electrostatic RESS.

The deposition methods described above allow controlled, micron thick layers of polymer or drug that can be deposited to form the layering patterns described above. Generating coatings and polymer films utilizing supercritical fluid technology circumvents the need to use and remove biologically detrimental organic solvents from coated biomaterials.

The release of pharmaceuticals into the host's body is controlled by the concentration of the agent, the layer thickness of the agent and the polymer and the kinetics of the sorption of the bio-absorbable polymer.

## Claims

1. A medical implant device comprising:
a device substrate having a surface;
a silane derivative coating covalently bonded to the surface;
a hetero-bifunctional polyethylene glycol spacer covalently bonded to the coating;
a bioactive agent covalently bonded to the spacer,
wherein the covalent bonding between the derivitized silane comprising terminal amine groups and hetero-bifunctional polyethylene glycol uses in a first condensation chemistry reaction carboxylated polyethylene glycol to produce a peptide bond between the derivitized silane and the heterobifunctional polyethylene glycol, and
wherein the bioactive agent is heparin or another protein or peptide, the covalent bonding between the heterobifunctional polyethylene glycol and the bioactive agent uses an amine group on the polyethylene glycol in a second condensation chemistry reaction to produce a peptide bond between the heterobifunctional polyethylene glycol and the bioactive agent.

2. The device of claim 1 wherein the small molecule is heparin.

3. The device of claim 1 wherein the surface is oxidized.

4. The device of claim 1 further comprising a bio-absorbable coating over the bioactive agent, and wherein the bio-absorbable coating comprises one or more polymers selected from the group consisting of poly(lactide-co-glycolide (PLGA); poly(dl-lactide) (DLPLA); poly(1-lactide) (LPLA); polyglycolide (PGA); poly(dioxanone) (PDO); poly(glycolide-co-trimethylene carbonate) (PGA-TMC); poly(Ilactide-co-glycolide) (PGA-LPLA); poly(dl-lactide-co-glycolide) (PGA-DLPLA); poly(Ilactide-co-dl-lactide) (LPLA-DLPLA); and poly(glycolide-co-trimethylene carbonate-codioxanone) (PDO-PGA-TMC).

5. The device of claim 4 wherein the bio-absorbable coating comprises at least one pharmaceutical compound.

6. A method of making a medical implant device of claim 1, comprising:
providing a device substrate having a surface;
reacting the surface with derivitized silane comprising terminal amine groups to form a silane coating over the surface, the coating being covalently bonded to the surface;
covalently bonding hetero-bifunctional polyethylene glycol to the derivitized silane of the silane coating uses a first condensation chemistry reaction to produce a peptide bond between the terminal amine groups of the derivitized silane and the heterobifunctional polyethylene glycol wherein the hetero-bifunctional polyethylene glycol comprises carboxylated polyethylene glycol;
covalently bonding a bioactive agent, wherein the bioactive agent is heparin or another protein or peptide, to the heterobifunctional polyethylene glycol using an amine group on the hetero-bifunctional polyethylene glycol and using a second condensation chemistry reaction to produce a peptide bond between the hetero-bifunctional polyethylene glycol and the bioactive agent and thereby forming a hetero-bifunctional polyethylene glycol spacer between the derivitized silane of the silane coating and the bioactive agent.

7. The method of claim 6 further comprising oxidizing the surface to produce an oxidized surface, and wherein the reacting the surface comprises reacting the oxidized surface.

8. The method of claim 6 wherein the bioactive agent is heparin.

9. The method of claim 6 wherein the bioactive agent is a protein and wherein the covalently bonding occurs in the presence of 1-ethyl-3-(3-dimethylaminopropyl) carbodiimide hydrochloride to produce the peptide bond between the derivitized silane and the protein.

10. The method of claim 6 further comprising forming a bio-absorbable coating over the bioactive agent, wherein the bio-absorbable coating comprises one or more polymer is selected from the group consisting of poly(lactide-co-glycolide (PLGA); poly(dl-lactide) (DLPLA); poly(1-lactide) (LPLA); polyglycolide (PGA); poly(dioxanone) (PDO); poly(glycolide-co-trimethylene carbonate) (PGA-TMC); poly(Ilactide-co-glycolide) (PGA-LPLA); poly(dl-lactide-co-glycolide) (PGA-DLPLA); poly(Ilactide-co-dl-lactide) (LPLA-DLPLA); and poly(glycolide-co-trimethylene carbonate-codioxanone) (PDO-PGA-TMC).

## Patentansprüche

1. Ein medizinisches Implantationsprodukt, umfassend:
ein Produktsubstrat mit einer Oberfläche;
eine Silanderivat-Beschichtung, die kovalent an die Oberfläche gebunden ist;
einen hetero-bifunktionalen Polyethylenglycol-Abstandhalter, der kovalent an die Beschichtung gebunden ist;
ein biologisch aktives Mittel, das kovalent an den Abstandhalter gebunden ist, wobei die kovalente Bindung zwischen dem derivatisierten Silan, welches terminale Amingruppen umfasst, und hetero-bifunktionalem Polyethylenglycol in einer ersten Kondensationschemie-Reaktion carboxyliertes Polyethylenglycol benutzt, um eine Peptidbindung zwischen dem derivatisierten Silan und dem hetero-bifunktionalen Polyethylenglycol zu erzeugen, und
wobei das biologisch aktive Mittel Heparin oder ein anderes Protein oder Peptid ist, die kovalente Bindung zwischen dem hetero-bifunktionalen Polyethylenglycol und dem biologisch aktiven Mittel eine Amingruppe an dem Polyethylenglycol in einer zweiten Kondensationschemie-Reaktion benutzt, um eine Peptidbindung zwischen dem hetero-bifunktionalen Polyethylenglycol und
dem biologisch aktiven Mittel zu erzeugen.

2. Das Produkt nach Anspruch 1, wobei das kleine Molekül Heparin ist.

3. Das Produkt nach Anspruch 1, wobei die Oberfläche oxidiert ist.

4. Das Produkt nach Anspruch 1, welches weiterhin eine biologisch resorbierbare Beschichtung über dem biologisch aktiven Mittel umfasst, und wobei die biologisch resorbierbare Beschichtung ein oder mehrere Polymere umfasst, die ausgewählt sind aus der Gruppe, bestehend aus Poly(lactid-co-glycolid) (PLGA); Poly(dl-lactid) (DLPLA); Poly(1-lactid) (LPLA); Polyglycolid (PGA); Poly(dioxanon) (PDO); Poly(glycolid-co-trimethylencarbonat) (PGA-TMC); Poly(l-lactid-co-glycolid) (PGA-LPLA); Poly(dl-lactid-co-glycolid) (PGA-DLPLA); Poly(l-lactid-co-dl-lactid) (LPLA-DLPLA); und Poly(glycolid-co-trimethylencarbonat-co-dioxanon) (PDO-PGA-TMC).

5. Das Produkt nach Anspruch 4, wobei die biologisch resorbierbare Beschichtung wenigstens eine pharmazeutische Verbindung umfasst.

6. Ein Verfahren zur Herstellung eines medizinischen Implantationsprodukts nach Anspruch 1, umfassend:
Bereitstellen eines Produktsubstrats mit einer Oberfläche;
Umsetzen der Oberfläche mit derivatisiertem Silan, das terminale Amingruppen umfasst, um eine Silanbeschichtung über der Oberfläche zu bilden, wobei die Beschichtung kovalent an die Oberfläche gebunden ist;
kovalentes Binden von hetero-bifunktionalem Polyethylenglycol an das derivatisierte Silan der Silanbeschichtung unter Verwendung einer ersten Kondensationschemie-Reaktion, um eine Peptidbindung zwischen den terminalen Amingruppen des derivatisierten Silans und dem hetero-bifunktionalen Polyethylenglycol zu erzeugen, wobei das hetero-bifunktionale Polyethylenglycol carboxyliertes Polyethylenglycol umfasst;
kovalentes Binden eines biologisch aktiven Mittels, wobei das biologisch aktive Mittel Heparin oder ein anderes Protein oder Peptid ist, an das hetero-bifunktionale Polyethylenglycol unter Verwendung einer Amingruppe an dem hetero-bifunktionalen Polyethylenglycol und Verwenden einer zweiten Kondensationschemie-Reaktion, um eine Peptidbindung zwischen dem hetero-bifunktionalen Polyethylenglycol und dem biologisch aktiven Mittel zu erzeugen, und dadurch Bilden eines hetero-bifunktionalen Polyethylenglycol-Abstandhalters zwischen dem derivatisierten Silan der Silanbeschichtung und dem biologisch aktiven Mittel.

7. Das Verfahren nach Anspruch 6, weiterhin umfassend das Oxidieren der Oberfläche, um eine oxidierte Oberfläche zu erzeugen, und wobei das Umsetzen der Oberfläche ein Umsetzen der oxidierten Oberfläche umfasst.

8. Das Verfahren nach Anspruch 6, wobei das biologisch aktive Mittel Heparin ist.

9. Das Verfahren nach Anspruch 6, wobei das biologisch aktive Mittel ein Protein ist und wobei die kovalente Bindung in der Gegenwart von 1-Ethyl-3-(3-dimethylaminopropyl)carbodiimid-Hydrochlorid stattfindet, um die Peptidbindung zwischen dem derivatisierten Silan und dem Protein zu erzeugen.

10. Das Verfahren nach Anspruch 6, weiterhin umfassend das Bilden einer biologisch resorbierbaren Beschichtung über dem biologisch aktiven Mittel, wobei die biologisch resorbierbare Beschichtung ein oder mehrere Polymere umfasst, die ausgewählt sind aus der Gruppe, bestehend aus Poly(lactid-co-glycolid) (PLGA); Poly(dl-lactid) (DLPLA); Poly(1-lactid) (LPLA); Polyglycolid (PGA); Poly(dioxanon) (PDO); Poly(glycolid-co-trimethylencarbonat) (PGA-TMC); Poly(l-lactid-co-glycolid) (PGA-LPLA); Poly(dl-lactid-co-glycolid) (PGA-DLPLA); Poly(l-lactid-co-dl-lactid) (LPLA-DLPLA); und Poly(glycolid-co-trimethylencarbonat-co-dioxanon) (PDO-PGA-TMC).

## Revendications

1. Dispositif implant médical, comprenant :
un substrat de dispositif comportant une surface ;
un revêtement à base de dérivé de silane lié de manière covalente à la surface ;
une couche d'espacement à base de polyéthylène glycol hétéro-bifonctionnel liée de manière covalente au revêtement ;
un agent bioactif lié de manière covalente à la couche d'espacement,
dans lequel la liaison covalente entre le silane dérivé comprenant des groupes amine terminaux et le polyéthylène glycol hétéro-bifonctionnel fait appel à un polyéthylène glycol carboxylé de première réaction chimique de condensation pour produire une liaison peptidique entre le silane dérivé et le polyéthylène glycol hétéro-bifonctionnel, et
dans lequel l'agent bioactif est de l'héparine ou un autre peptide ou protéine, la liaison covalente entre le polyéthylène glycol hétéro-bifonctionnel et l'agent bioactif fait appel à un groupe amine sur le polyéthylène glycol lors d'une seconde réaction chimique de condensation pour produire une liaison peptidique entre le polyéthylène glycol hétéro-bifonctionnel et l'agent bioactif.

2. Dispositif selon la revendication 1, dans lequel la petite molécule est de l'héparine.

3. Dispositif selon la revendication 1, dans lequel la surface est oxydée.

4. Dispositif selon la revendication 1, comprenant en outre un revêtement bio-absorbable sur l'agent bioactif, et dans lequel le revêtement bio-absorbable comprend un ou plusieurs polymères choisis dans le groupe constitué par le poly(lactide-co-glycolide) (PLGA) ; le poly(dl-lactide) (DLPLA) ; le poly(1-lactide) (LPLA) ; le polyglycolide (PGA) ; la poly(dioxanone) (PDO) ; le poly(glycolide-co-triméthylène carbonate) (PGA-TMC) ; le poly(Ilactide-co-glycolide) (PGA-LPLA) ; le poly(dl-lactide-co-glycolide) (PGA-DLPLA) ; le poly(Ilactide-co-dl-lactide) (LPLA-DLPLA) ; et la poly(glycolide-co-triméthylène carbonate-codioxanone) (PDO-PGA-TMC).

5. Dispositif selon la revendication 4, dans lequel le revêtement bio-absorbable comprend au moins un composé pharmaceutique.

6. Procédé de fabrication d'un dispositif implant médical selon la revendication 1, comprenant :
la fourniture d'un substrat de dispositif comportant une surface ;
la réaction de la surface avec un silane dérivé comprenant des groupes amine terminaux pour former un revêtement à base de silane sur la surface, le revêtement étant lié de manière covalente à la surface ;
la liaison de manière covalente d'un polyéthylène glycol hétéro-bifonctionnel avec le silane dérivé du revêtement de silane en faisant appel à une première réaction chimique de condensation pour produire une liaison peptidique entre les groupes amine terminaux du silane dérivé et le polyéthylène glycol hétéro-bifonctionnel, dans lequel le polyéthylène glycol hétéro-bifonctionnel comprend un polyéthylène glycol carboxylé ;
la liaison de manière covalente d'un agent bioactif, dans lequel l'agent bioactif est de l'héparine ou un autre peptide ou protéine, avec le polyéthylène glycol hétéro-bifonctionnel en faisant appel à un groupe amine sur le polyéthylène glycol hétéro-bifonctionnel et à une seconde réaction chimique de condensation pour produire une liaison peptidique entre le polyéthylène glycol hétéro-bifonctionnel et l'agent bioactif et former ainsi une couche d'espacement à base de polyéthylène glycol hétéro-bifonctionnel entre le silane dérivé du revêtement de silane et l'agent bioactif.

7. Procédé selon la revendication 6, comprenant en outre l'oxydation de la surface pour produire une surface oxydée, et dans lequel la réaction de la surface consiste à faire réagir la surface oxydée.

8. Précédé selon la revendication 6, dans lequel l'agent bioactif est de l'héparine.

9. Procédé selon la revendication 6, dans lequel l'agent bioactif est une protéine et dans lequel la liaison covalente se produit en présence du 1-éthyl-3-(3-diméthylaminopropyl) carbodiimide hydrochlorure pour produire la liaison peptidique entre le silane dérivé et la protéine.

10. Procédé selon la revendication 6, comprenant en outre la formation d'un revêtement bio-absorbable sur l'agent bioactif, dans lequel le revêtement bio-absorbable comprend un ou plusieurs polymères choisis dans le groupe constitué par le poly(lactide-co-glycolide) (PLGA) ; le poly(dl-lactide) (DLPLA) ; le poly(1-lactide) (LPLA) ; le polyglycolide (PGA) ; la poly(dioxanone) (PDO) ; le poly(glycolide-co-triméthylène carbonate) (PGA-TMC) ; le poly(Ilactide-co-glycolide) (PGA-LPLA) ; le poly(dl-lactide-co-glycolide) (PGA-DLPLA) ; le poly(Ilactide-co-dl-lactide) (LPLA-DLPLA) ; et la poly(glycolide-co-triméthylène carbonate-codioxanone) (PDO-PGA-TMC).
